# EUROPEAN PATENT APPLICATION

(11) **EP 1 839 663 A1**
(43) Date of publication of application: **03.10.2007**
(21) Application number: 05822703.4
(22) Date of filing: 27.12.2005
(51) Int. Cl.: A61K 31/7016, A61K 31/352, A61P 3/06, A61P 3/14, A61P 7/10, A61P 9/10, A61P 43/00, A23K 1/16, A23K 1/18, A23L 1/30

(54) **BLOOD CHOLESTEROL REDUCING ORAL COMPOSITION**

(30) Priority: 12.01.2005 JP 2005005738
(71) Applicant: Fancl Corporation, Kanagawa 231-0806 (JP)
(72) Inventor: TAMURA, A., Fancl Res. Institute, Fancl Corp., Yokohama-shi, Kanagawa 2440806 (JP); SHIGEMATSU, N., Fancl Corp., Yokohama-shi, Kanagawa 2310806 (JP); HARA, H., Hokkaido Univ. Graduate School Agricult., Sapporo-shi, Hokkaido 0608589 (JP)
(74) Representative: Vossius & Partner
(86) International application number: PCT/JP2005/023880
(87) International publication number: WO 2006/075523

(57) **Abstract**

A composition capable of remedying hypercholesterolemia through enhancing of the blood total cholesterol reducing potency of isoflavone. There is provided a blood cholesterol reducing oral composition, comprising difructose anhydride and isoflavone and/or its derivative as an active ingredient.

## Description

### Field of the Invention

The present invention relates to a composition for lowering blood cholesterol.

### Background Technology

According to a national survey on nutrition in Japan conducted in 2002, it is revealed that the total serum cholesterol level exceeds 240 mg/dL in 11.5% of males and 18.2% of females, which suggests the presence of a considerable number of hypercholesterolemic patients. It has been known that the relative risk of developing coronary arterial diseases consecutively increases as the level of the total serum cholesterol increases (Clinical Guideline for the Treatment of Arteriosclerotic Diseases, 2002 edition, the Japan Arteriosclerosis Society). Under these circumstances, not only a drug therapy but also a therapy by improving lifestyle including diet has been tried.
Isoflavones are known to be effective in treating hypercholesterolemia (Patent Reference 1: Japanese Patent Application Laid-open No. 1997-255570). Further, a method of reducing a risk of developing arteriosclerosis by reducing LDL-cholesterol and increasing HDL-cholesterol using daidzein, one of isoflavones, has been reported (Patent Reference 2: Japanese Patent Application Laid-open No. 1999-139973). However, the number of people with hypercholesterolemia has kept increasing even in Japan where consumption of soybeans having a high isoflavone content is high, which suggests that the effect of isoflavones is not necessarily sufficient from a social viewpoint.
As for difructose anhydride (referred to as DFA hereinafter), a kind of oligosaccharide, it is known to have a growth promoting effect on bifidobacteria (Patent Reference 3: Japanese Patent Publication No. 1991-5788), to increase the amount of calcium absorption (Patent Reference 4: Japanese Patent No. 3514955), and to have a diuretic effect (Patent Reference 5: Japanese Patent Application Laid-open No. 2003-321371); however, its cholesterol lowering effect in combination with isoflavones has not been known. On the other hand, as for oligosaccharides, an agent for improving hyperglycemia in which fine dietary fiber and oligosaccharides are combined has been invented and fructooligosaccharides are particularly known to be effective (Patent Reference 6: Japanese Patent Application Laid-open No. 2000-154143).

Patent Reference 1: Japanese Patent Application Laid-open No. 1997-255570
Patent Reference 2: Japanese Patent Application Laid-open No. 1999-139973
Patent Reference 3: Japanese Patent Publication No. 1991-5788
Patent Reference 4: Japanese Patent No. 3514955
Patent Reference 5: Japanese Patent Application Laid-open No. 2003-321371
Patent Reference 6: Japanese Patent Application Laid-open No. 2000-154143

### DISCLOSURE OF THE INVENTION

### Problems that the Invention Attempts to Solve

An object of the present invention is to provide a composition for improving hypercholesterolemia by augmenting a total blood cholesterol lowering effect of isoflavones.

### Means to Solve the Problems

In the course of intensive studies to develop a means for augmenting the total blood cholesterol lowering effect of isoflavones, the present inventors have found that the total blood cholesterol lowering effect of isoflavones is augmented by DFA ingestion. Thus, the present invention is a composition which exhibits a blood cholesterol lowering effect by using DFA and an isoflavone in combination.

Namely, the present invention is primarily composed of an oral composition for lowering blood cholesterol, which is characterized in that it augments the cholesterol lowering effect of isoflavones by containing difructose anhydride and an isoflavone and/or its derivative as active ingredients.
(1) An oral composition for lowering blood cholesterol, characterized in that it contains difructose anhydride and an isoflavone and/or its derivative as active ingredients.
(2) An oral composition for lowering blood cholesterol, characterized in that it contains difructose anhydride and daidzein and/or its derivative as active ingredients.
(3) An oral composition for lowering blood cholesterol characterized in that it augments a cholesterol lowering effect of an isoflavone by containing difructose anhydride and an isoflavone and/or its derivative as active ingredients.
(4) An oral composition for lowering blood cholesterol, characterized in that it augments a cholesterol lowering effect of daidzein by containing difructose anhydride and daidzein and/or its derivative as active ingredient.
(5) A pharmaceutical or food product containing the composition described in any one of (1) to (4).
(6) A pharmaceutical product for pet animals or a feed product for pet animals containing the composition described in any one of (1) to (4).

### Effectiveness of the Invention

A compound of the present invention containing DFA and an isoflavone and/or its derivative has a blood cholesterol lowering effect.

### BEST MODE FOR CARRYING OUT THE INVENTION

In the present invention, DFA refers to an anhydrized cyclic disaccharide, a known substance, in which two fructose units are linked to one another each at two points. While it has been conventionally known to be present in caramel and the like, it can industrially be manufactured by the fermentation of inulin with an inulin hydrolyzing enzyme such as inulin fructotransferase (EC2.4.1.93) produced by Arthrobacter sp. H65-7 strain or by the fermentation of levan with levan fructotransferase (EC2.4.1.1 10) produced by Arthrobacter nicotinovorans GS-9. There are five derivatives with different modes of linkage between two fructose molecules; they are called DFA I, DFA II, DFA III, DFA IV, and DFA V, respectively. Although DFA according to the present invention refers to all of them, DFA III and DFA IV are more preferably used in the present invention because of their excellence in terms of efficiency in industrial production, stability after purification, and the like.

An oral composition according to the present invention comprises DFA as an active ingredient and can be used as a composition for pharmaceutical products or food and drink type products (including feed products for pet animals). The forms of the products are not particularly limited as long as they can ultimately be used for oral administration, and include pharmaceutical products for humans or pet animals, food and drink products, powdered milk formulations, enteral feeding formulations, health foods and drinks, and additives for feed products. The most preferred form used in the present invention is a form in which DFA and an isoflavone and/or its derivative reach the large intestine without being absorbed and, for example, they are contained in an enteric capsule. Further, the content of an active ingredient is not particularly limited.

When used as a food or drink type composition, DFA can be appropriately used as it is or in combination with other food products or food components according to an ordinary method. Further, upon processing, a conventional method for processing food products can be applied without difficulty because of its excellent heat stability and acid stability. The form of a food type oral composition containing DFA is not particularly limited and can be a form of powder, granule, paste, liquid, suspension, tablet, capsule, or the like. For example, it can be formulated into a health drink using sweetening agents, food acidulants, vitamins, and various other ingredients used for manufacturing energy drinks.

When used as a pharmaceutical composition, this active ingredient can be administered in various modes. An example of the mode of administration is an oral administration in the form of tablets, capsules, granules, dispersal powder, syrup, or the like. These various formulations can be formulated according to a conventional method by adding known auxiliaries generally used in the technological field of medicinal preparation, such as excipients, binders, disintegrators, lubricants, flavoring agents, dissolution agents, suspending agents, and coating agents, to a main ingredient.
The amount to be used varies depending on symptoms, age, body weight, and drug form; however, the amount of daily administration for an adult ranges from 0.5 mg to 2000 mg, preferably from 1 mg to 1000 mg, per kg body weight.

Further, an isoflavone in the present invention refers to a component having an isoflavone structure, including generally called genistein, daidzein, and glycitin, and its derivative refers to its glycoside and/or its derivative modified by acetylation, malonylation, or the like. In the present invention, daidzein, which can be transformed by intestinal bacteria into equol having a higher activity, and/or its derivative are preferably used. An isoflavone and/or its derivative used in the present invention can be derived from a chemically synthesized product, extract of a natural product and its processed product, or a plant itself.

The present invention will be explained in more detail by the following example that is for exemplary purposes only and is not intended as a limitation of the invention.

### Example 1

A total plasma cholesterol lowering effect was studied using DFA and an isoflavone in combination.
After preliminary feeding 21 VVister-ST male rats (6 weeks of age) a non-isoflavone feed for 3 days, the animals were divided into 3 groups (each n=7) by a body weight stratification procedure and were individually fed for 19 days a basal feed containing 0.25% food product supplemented with 40% isoflavone (basal diet group), a feed in which cellulose in the basal feed was replaced with 1.5% (final) DFA III (DFA III group), and a feed in which cellulose in the basal feed was replaced with 1.5% (final) fructooligosaccharide (FOS group), as shown in Table 1. On the 20th day, blood samples were taken from the abdominal aorta to obtain plasma by centrifugation and the total plasma cholesterol was measured using an automatic analyzer (DRI-CHEM 3500, Fuji Film Medical).

The results are shown in Table 2. In the table, figures with different alphabets, a and b, denote that there is a significant difference between the figures. Significant difference according to the Sheffe method was p=0.051 between the basal diet group and the DFA III group, which suggested a strong tendency. The level of HDL cholesterol was slightly low in the DFA III group; however, the differences between the groups were not significant.
The results evidently show that the total serum cholesterol level tended to be lowered by DFA ingestion as compared to the group fed an isoflavone alone. This effect was not observed with a fructooligosaccharide, a known oligosaccharide having a hyperglycemia improving effect.

**Table 1**

| | Basal diet | DFA III diet | FOS diet |
|---|---|---|---|
| Casein | 200 | 200 | 200 |
| Dextrin | 453 | 453 | 453 |
| Corn oil | 70 | 70 | 70 |
| Mineral mix | 35 | 35 | 35 |
| Vitamin mix | 10 | 10 | 10 |
| Choline bitartate | 2.5 | 2.5 | 2.5 |
| L-cystine | 3 | 3 | 3 |
| Anti-oxidant | 0.014 | 0.014 | 0.014 |
| Cellulose | 80 | 65 | 65 |
| Isoflavone (40%) | 2.5 | 2.5 | 2.5 |
| DFA III | 0 | 15 | 0 |
| FOS | 0 | 0 | 15 |
| Sucrose | 143.986 | 143.986 | 143.986 |
| Total (g) | 1000 | 1000 | 1000 |

**Table 2**

| | Basal diet | DFA III 1.5% | FOS 1.5% |
|---|---|---|---|
| Total cholesterol (mg/L) | 7.6±0.5 ab | 6.1±0.3 a | 7.7±0.4b |
| HDL-cholesterol (mg/L) | 4.2±0.3 | 3.7±0.4 | 4.1±0.3 |

## Claims

1. An oral composition for lowering blood cholesterol, **characterized in that** it contains difructose anhydride and an isoflavone and/or its derivative as active ingredients.

2. An oral composition for lowering blood cholesterol, **characterized in that** it contains difructose anhydride and daidzein and/or its derivative as active ingredients.

3. A pharmaceutical or food product containing the composition described in claim 1 or 2.

4. A pharmaceutical product for pet animals or a feed product for pet animals containing the composition described in claim 1 or 2.
